(19) Europäisches Patentamt

European Patent Office

Office européen des brevets

(11) Veröffentlichungsnummer: **0 302 186 B1**

(12) **EUROPÄISCHE PATENTSCHRIFT**

(45) Veröffentlichungstag der Patentschrift: **13.05.92**

(51) Int. Cl.⁵: **A61L 29/00**, A61L 27/00

(21) Anmeldenummer: **88107039.5**

(22) Anmeldetag: **03.05.88**

(54) **Medizinisches Gerät und Verfahren zu seiner Herstellung.**

(30) Priorität: **04.08.87 DE 3725728**

(43) Veröffentlichungstag der Anmeldung:
**08.02.89 Patentblatt  89/06**

(45) Bekanntmachung des Hinweises auf die
Patenterteilung:
**13.05.92 Patentblatt  92/20**

(84) Benannte Vertragsstaaten:
**AT BE CH DE ES FR GB GR IT LI LU NL SE**

(56) Entgegenhaltungen:
**EP-A- 0 068 385          EP-A- 0 190 504
EP-A- 0 206 024          EP-A- 0 207 624
US-A- 3 562 352          US-A- 4 054 139
US-A- 4 581 028          US-A- 4 592 920**

(73) Patentinhaber: **Firma Carl Freudenberg
Höhnerweg 2-4
W-6940 Weinheim/Bergstrasse(DE)**

(72) Erfinder: **Brenner, Otto
Bismarckstrasse 38
W-6803 Edingen(DE)**
Erfinder: **Ermert, Wolfgang, Dr.
Ahornstrasse 88
W-6940 Weinheim(DE)**
Erfinder: **Eschwey, Helmut, Dr.
Uhlandstrasse 25
W-6946 Gorxheimertal(DE)**
Erfinder: **Esswein, Gerd, Dr.
Kurpfalzstrasse 78
W-6701 Maxdorf(DE)**
Erfinder: **Schuhmacher, Günter, Dr.
Steingasse 11
W-6940 Weinheim(DE)**
Erfinder: **Thaler, Martin, Dr.
Kapplerbergstrasse 22
W-7753 Allensbach 1(DE)**

**Beschreibung**

Die vorliegende Erfindung betrifft ein medizinisches Gerät nach dem Oberbegriff des Anspruchs 1 sowie ein Verfahren zu seiner Herstellung.

Die in der Literatur (z.B.: The Journal of Urology, 121 <Januar 1979>, 40 ff. ) häufig als oligodynamisch bezeichnete Mikrobizidwirkung von Schwermetallionen, wie Gold, Silber oder Kupfer, wird in der Medizintechnik genutzt, um Kunststoff-Endoprothesen oder schlauchförmige Katheter, wie z. B. Harnröhrenkatheter, für eine längere Zeitdauer mikrobizid auszurüsten.

Die US-PS 4,054,139 schlägt hierzu vor, einen röhrenförmigen Katheter auf seiner Innen- und auf seiner Außenfläche mit mindestens einem oligodynamischen Agens, z. B. Silber oder seinen Verbindungen, zu versehen.

Um die Oberfläche eines solchen medizinischen Gerätes in quantitativer Hinsicht noch aufnahmefähiger für Metallionen abgehende Substanzen zu machen, wird in der US-PS 4,612,337 gelehrt, das Polymermaterial, z. B. ein Polyurethan oder Silikon-Elastomer, durch zweimaliges Tränken bzw. Quellen mit geeigneten Lösungsmitteln (für das Mikrobizid bzw. für das Metallsalz) zu behandeln und jeweils anschließend, zuletzt nach dem Auswaschen, zu trocknen.

Unbefriedigend ist bei allen bekannten Lösungen, bei denen eine innen- und außenseitige mikrobizide Ausrüstung des Gerätes erwünscht ist, der hohe Arbeitsaufwand des Beschichtens der Oberflächen.

Da ferner die Metallionen abgebenden Substanzen stets nur in den Oberflächenbereichen des Polymerwerkstoffes gebunden vorliegen, ist ihre Verfügbarkeit zeitlich und mengenmäßig begrenzt, was bei Langzeitbehandlungen einen häufigen, risikoreichen und schmerzhaften Austausch mit neu beschichteten Geräten erforderlich macht.

Für alle nicht flächig aufgebauten, sondern eine Raumform aufweisenden, intracorporal zu verwendenden Geräte, z. B. Katheter oder Prothesen, stößt man bereits bei etwas größeren Materialdicken an die Grenzen der Einlagerungs- oder Abgabefähigkeit für Metallionen abgebende Substanzen: Im Falle von Silikon-Elastomeren scheitert man daran, daß die inneren Bereiche dieses Materials wegen ihrer Hydrophobie für Körperflüssigkeit nicht oder nicht in ausreichendem Maße zugänglich sind. Dies bedeutet jedoch nichts anderes, als daß den tiefer in die Matrix eingebetteten Ionen kein Trägermedium mehr zur Verfügung steht, mittels dessen sie wieder nach außen wandern könnten, um ihre mikrobizide Wirkung in dem den Katheter umgebenden Körpergewebe zu entfalten. Es sind somit nach wie vor lediglich die an der Oberfläche befindlichen Agenzien wirksam.

Die Alternative, die Matrix für die Metallionen abgebenden Substanzen aus Polyurethan zu fertigen, läßt zwar die reversible Einbettung dieser Substanzen bis in innerste Werkstoffzonen zu, da Polyurethan ausreichend hydrophil auch in seinem Innern ist und unter Einwirkung von Körperflüssigkeit somit durchaus Metallionen abgeben könnte. Solchermaßen aufgebaute Geräte jedoch weisen, wie Messungen ergaben, derart niedrige Abgaberaten für oligodynamisch wirksame Substanzen auf, daß eine, gemessen an der Konzentration der zu vernichtenden Keime, ausreichende Menge an wirksamen Ionen bei Daueranwendung nicht zur Verfügung steht.

Aus der EP-PS 0 068 385 ist die ausgeprägt antithrombogene Wirkung eines thermoplastischen Polyurethan-Silikon-Elastomerwerkstoffes und dessen vorteilhafte Verwendung für medizinische Formteile, die unmittelbar mit Blut in Kontakt gebracht werden, bekannt geworden. Es werden dort aus der US-PS 3,562,352 vorbekannte Block-Copolymere aus einem Polyurethan und einem organischen Silikonpolymeren mit weniger Silikon- als Polyurethan-Einheiten bezüglich ihrer mechanischen sowie antithrombogenen Eigenschaften bei unmittelbarem Blutkontakt diskutiert sowie eine neuartige, vorteilhafte Variante ausführlich beschrieben: Bei dieser sind in die Polymer-Hauptkette 4 bis 15 Gew.-% eines organischen Silikonpolymeren mit einem Molekulargewicht von 500 bis 10.000 und weichmachende Polyether- oder Polyester-Segmente eingebracht. Auch dieser Werkstoff enthält keine medizinisch wirksamen, nach außen abzugebenden Wirkstoffe; ein Hinweis auf seine eventuelle Verwendbarkeit für mikrobizid wirkende, nicht mit Blut in Verbindung stehende medizinische Geräte ist, wie auch in der genannten Europäischen Patentschrift, nicht enthalten.

US-PS 4,612,337 beschreibt ein Verfahren, bei dem ein in der Medizin verwendbarer polymerer Substratkörper, bevorzugt PTFE, zweimal getränkt wird mit zwei verschiedenen antimikrobiellen Agenzien, welche jeweils in organischen Lösungsmittelgemischen gelöst sind. Anhand von Versuchsreihen wird gezeigt, daß dabei jede Behandlung der polymeren Substrate allein mit Silbersalzlösung, ohne Zugabe von Tridodecylmethylammoniumchlorid, zu keiner antibakteriellen Aktivität führt.

Der Erfindung liegt die Aufgabe zugrunde, ein im lebenden Körper zu verwendendes, Körpergewebe durchdringendes medizinisches Gerät anzugeben, welches eine wesentlich erhöhte Abgabekapzität für mikrobizide, Metallionen abgebende Substanzen und eine gute Gewebeverträglichkeit aufweist und welches die Metallionen in kontrollierter Menge über einen längeren Zeitraum zu jeder seiner Außenseiten wieder abzugeben imstande ist, wenn es mit Körperflüssigkeit in Berührung kommt. Zugleich soll ein Verfahren zur Herstellung eines solchen medizinischen Gerätes aufgezeigt werden, wobei, ohne Nachbehandlung des Werkstoffes oder des Fertigteils durch umständliche mechanische oder chemische Verfahren, eine räumlich gleichmäßige Einbettung einer Metallionen abgehende Substanz in feinstverteilter Form in die Polymermatrix bereits während deren Herstellung möglich ist. Die bisherige Erfordernis der Zugabe von synergistischen Substanzen sowie von Lösunmgsmitteln soll entfallen.

Die Lösung dieser Aufgabe besteht in einem medizinischen Gerät mit den kennzeichnenden Merkmalen des Anspruch 1. Besonders bevorzugte Herstellungsverfahren für ein gattungsgemäßes Gerät werden in den Ansprüchen 2 und 3 dargelegt.

Die für das Gerät erforderliche Werkstoffklasse wird im folgenden "Silikonpolyurethan" genannt. Herstellung und Eigenschaften dieses Werkstoffes sind für die vorliegende Erfindung nicht kritisch und je nach den gewünschten Anforderungen ohne erfinderisches Zutun im Rahmen der beanspruchten Lehre aus dem Stand der Technik herleitbar. So können in bekannter Weise, je nach den für zweckmäßig erachteten Materialeigenschaften, weichmachende Polyether- oder Polyester-Segmente, Kettenverlängerer als thermoplastische bzw. Kettenverlängerer und/oder Vernetzungsmittel als duroplastische Eigenschaften verleihende Zusätze verwendet werden.

Als Metallionen abgebende, mikrobizide, oligodynamisch wirksame Substanz werden z.B. Metallsalze, deren Oxide und Carbide sowie metallorganische Verbindungen verstanden, wie sie in der Medizin bezüglich Wirkung und Anwendung wohlbekannt sind. Eine solche Substanz ist erfindungsgemäß ohne Abmischung mit weiteren synergistischen Mikrobiziden in der Polymermatrix vorhanden.

Die erfindungsgemäß 1- bis 15prozentige räumliche, gleichmäßige Einbettung der aktiven Substanz in die Silikonpolyurethan-Matrix erhöht überraschenderweise die nutzbare Menge der zur Verfügung stehenden Ionen gegenüber den üblichen Beschichtungen um 40 % und mehr.

Überraschend war auch, daß der Kontakt von Körperflüssigkeit mit den inneren Bereichen des bisher nur für andere Zwecke als vorteilhaft bekannten Silikonpolyurethan-Werkstoffes sicher gewährleistet ist, auch wenn die Polymermatrix für gattungsgemäße Geräte übliche Dicken von bis zu 2 mm aufweist.

Die Erfindung ist vorzugsweise, jedoch nicht ausschließlich, dann anzuwenden, wenn die betreffenden Geräte nicht nur vorübergehend, sondern länger im Körpergewebekontakt verbleiben müssen; sie umfaßt z.B. Endoprothesen, Katheter, Sonden, Endoskope, Implantate und Dränagen.

Da die das Gerät bildende polymere Matrix aus Silikonpolyurethan erfindungsgemäß zur Gänze den oligodynamischen Wirkstoff enthält, tritt, neben dem Vorteil der Abgabe der Metallionen zu beiden Außenflächen des Gerätekörpers hin, noch der vorteilhafte Effekt auf, daß zu Beginn der Anwendung, wo eine erhöhte Zahl von Bakterien, Mikroben und Keimen anwesend sein kann, eine erhöhte Menge an Wirkstoff mit abgegeben wird, sobald alle Oberflächenbereiche der Matrix mit Körperflüssigkeit durchtränkt sind. Nach längerer Verweildauer steht ein stetig sich verminderndes Angebot an Metallionen zur Verfügung, deren Abgabe nunmehr von ihrer Weglänge bei der Wanderung aus dem Inneren der Matrix an deren Oberfläche bestimmt wird.

Zu diesem Zeitpunkt muß aber auch nur noch gewährleistet sein, daß die - geringe - Anzahl potentiell neu hinzukommender Keime sofort abgetötet wird. Die erfindungsgemäße Ausgestaltung des medizinischen Gerätes paßt somit die Abgaberate an Metallionen der Menge der momentan zu bekämpfenden Organismen von selbst an.

Zweckmäßigerweise sollte die Metallionen abgebende Substanz in der Matrix keine Korngrößen größer als 50 $\mu$m enthalten, da ein Überschreiten dieser Grenze die mechanische Stabilität der Matrix negativ beeinflussen kann.

Bei erfindungsgemäß ausgestalteten Harnröhrenkathetern wurde außerdem der vorteilhafte Effekt beobachtet, daß Inkrustationen am Katheter, d.h. Überkrustungen mit Mineralien, in deutlich vermindertem Maße oder gar nicht auftraten.

Ein bevorzugtes Verfahren zur Herstellung des erfindungsgemäßen Gerätes besteht darin, daß man das Polyol, das organische Silikonpolymer sowie das Isocyanat sowie gegebenenfalls die Kettenverlängerer und/oder Vernetzersubstanzen miteinander reagieren läßt und danach in die gewünschte Form bringt.

Erfindungsgemäß wird nun direkt während dieser Reaktion die Metallionen abgebende Substanz mit einem Anteil an der Gesamtpolymermasse von 1- bis 15 Gew.-% als Pulver mit einer Korngröße $\leq$ 50 $\mu$m vor der Zugabe des Isocyanats zugemischt und gemeinsam mit dem Polyol entwässert. Durch diese Verfahrensabfolge und die Feinkörnigkeit der wirksamen Substanz bei der Zugabe werden folgende Vorteile erzielt:

Die getrennten Arbeitsgänge des Trocknens der Metallionen abgebenden Substanz sowie des Polyols und/oder des Polyolgemisches werden zu einem einzigen Verfahrensschritt zusammengefaßt. Die Feinkör-

nigkeit garantiert die Möglichkeit, die aktive Substanz über den gesamten Querschnitt der Polymermatrix besonders gleichmäßig einzubringen. Der Einsatz eines organischen Lösungsmittel für die aktive Substanz sowie das bisher notwendige, mehrmalige Tränken des fertigen Werkstoffes mit der Wirkstofflösung sind nicht mehr erforderlich.

In einem Alternativverfahren wird eine gut wasserlösliche, Metallionen abgebende Substanz als wäßrige Lösung mit einem Feststoffanteil wie beim ersten Verfahren und an gleicher Stelle wie dort der Polymermasse zugemischt, anschließend das Ganze entwässert und sodann das Isocyanat zugegeben. Ebenfalls ist dabei kein Lösungsmittel vonnöten. Weiter besteht der Vorteil, die aktive Substanz in kleinstmöglicher Form, nämlich als diskrete Ionen und damit optimal gleichmäßig, in die Matrix einführen zu können.

Das anschließende Entwässern führt zu keinen größeren Kristall-Agglomeraten, da die polymere Matrix nur die Entstehung kleiner Kristallite ( $\leq$ 50 $\mu$m) zuläßt. Zweckmäßig wird dabei als oligodynamisch aktive Substanz Silbernitrat eingesetzt.

Die erfindungsgemäßen Verfahren besitzen weiterhin folgende Vorteile:
Es wird eine einstufige Formgebung und bakterizide/mikrobizide Ausrüstung des medizinischen Gerätes erzielt. Jegliche Nachbehandlung entfällt, wodurch das Handhaben von Lösungsmitteln, das Quellen oder Anlösen des Substrates oder Probleme bezüglich der Haftung von Beschichtungen vollständig umgangen werden können.

Will man einen thermoplastischen Werkstoff erhalten, wird das fertig hergestellte, mit Wirkstoff ausgerüstete Polyurethan granuliert, getrocknet und anschließend entweder spritzgegossen oder extrudiert. Für die Herstellung von duroplastischen Geräten, z. B. Sonden, erfolgt die Formgebung sofort nach der Reaktion der Ausgangsstoffe durch Ausgießen in ein Formteil.

In jedem Falle kann das gewünschte medizinische Gerät in einem kontinuierlich verlaufenden Verfahrensschritt hergestellt werden.

Anhand des folgenden Beispieles wird in nicht einschränkender Weise ein Herstellungsverfahren für ein medizinisches Gerät aus einem thermoplastischen Werkstoff detailliert beschrieben:
100 Teile eines OH-terminierten, bifunktionellen Polyesters mit dem Molekulargewicht 2.000 g/mol (Basis: Adipinsäure, Neopentylglykol plus Hexandiol), wie er sich unter der Bezeichnung Desmophen 2028 der Bayer AG im Handel befindet, werden mit 20 Teilen eines linearen, endständige Hydroxylgruppen aufweisenden Polydimethylsiloxans (Molekulargewicht 2.000 g/mol) und 6 Teilen pulverisierten Silbernitrats (Korngröße $\leq$ 50 $\mu$m) gemischt und eine Stunde bei 120° C im Vakuum entwässert. Danach wird das entstandene Präpolymer mit 40 Teilen Diphenylmethan-4,4'-diisocyanat gemischt und nach dem Ausreagieren mit 9 Teilen Butandiol die Polymerkette verlängert.

Das fertige Produkt wird auf der Heizplatte aushärten gelassen, anschließend granuliert und zwei Stunden bei 110°C in einem Warmluftofen getrocknet. Zum Schluß wird das Granulat auf einer Spritzgießmaschine in die Form eines Harnröhrenkatheters gebracht.

Der so gefertigte Katheter besitzt die folgenden Materialeigenschaften:

Zugfestigkeit (DIN 53 504)         : 20 MPa
Bruchdehnung (DIN 53 504)          : 500%
Härte (DIN 53 305)                 : 70 Shore A

Diese Materialdaten zeigen die mechanische Eignung des thermoplastischen Werkstoffes für die Herstellung von Kathetern und Implantaten.

Die folgende Tabelle zeigt die mikrobizide Wirkung sowie die Abgabefähigkeit an oligodynamisch wirkenden Silberionen für eine Polyurethanmatrix gemäß dem Stand der Technik im Vergleich mit einer Silikonpolyurethan-Matrix gemäß der vorliegenden Erfindung:

| Test | Matrix mit Ag$^+$ aus: | |
| --- | --- | --- |
| | Polyurethan (St. d. Technik) | Silikonpolyurethan (wie Beispiel; Erfindung) |
| Ag$^+$-Diffusion $\langle$mg/l$\rangle$*) | 0,57 | 0,89 |
| mikrobiologische Plattendiffusion nach **) | | |
| 2 Tagen | + + + + | + + + + |
| 4 " | + + + + | + + + + |
| 8 " | + + | + + + + |
| 16 " | + | + + + + |
| 32 " | kein Hemmhof | + + + + |

*) Bestimmung der Silberionenkonzentration in Synthese-urin mittels Atomabsorptionsspektral-Analyse

**) Qualitative Beobachtung des jeweiligen Hemmhof-Durch-messers in Nährmedium mit pathogenen Keimen $\langle$Klebsiella oxytoca ATCC 33496; Streptococcus faecalis ATCC 29200; Enterobacter cloacae ATCC 29006 und Staphylococcus epi-dermidis$\rangle$, bezogen auf den maximalen Hemmhof-Durchmes-ser (+ + + +).

**Patentansprüche**

1. In das Körpergewebe einzuführendes, bei Kontakt mit Körperflüssigkeit bakterizide bzw. mikrobizide Metallionen abgebendes, gewebeverträgliches medizinisches Gerät, bestehend aus einem Polyurethan-Elastomer, in dessen Hauptkette zu weniger als 50 Gew.-% ein organisches Silikonpolymeres mit einem Molekulargewicht von 500 bis 10.000 vorhanden ist, und aus einer Metallionen abgebenden Substanz, dadurch gekennzeichnet, daß die Metallionen abgebende Substanz über den gesamten Querschnitt des Elastomeren in räumlich gleichmäßiger Verteilung und in Mengen von 1 bis 15 Gew.-%, bezogen auf die Gesamtmasse des Elastomeren, eingelagert ist.

2. Verfahren zur Herstellung eines in das Körpergewebe einzuführenden, bei Kontakt mit Körperflüssigkeit bakterizide bzw. mikrobizide Metallionen abgebenden, gewebeverträglichen medizinischen Gerätes, bestehend aus einem Polyurethan-Elastomeren, in dessen Hauptkette zu weniger als 50 Gew.-% ein organisches Silikonpolymer mit einem Molekulargewicht von 500 bis 10.000 vorhanden ist, und aus einer Metallionen abgebenden Substanz, wobei man das Polyol, das organische Silikonpolymer sowie das Isocyanat miteinander reagieren läßt und danach in die gewünschte Form bringt, dadurch gekennzeichnet, daß man vor der Zugabe des Isocyanats eine Metallionen abgebende Substanz mit einem Anteil an der Gesamtpolymermasse von 1 bis 15 Gew.-% als Pulver mit einer Korngröße ≦ 50 μm in räumlich gleichmäßiger Verteilung zumischt und gemeinsam mit dem Polyol entwässert, ohne daß eine mechanische oder chemische Nachbehandlung erfolgt.

3. Verfahren zur Herstellung eines in das Körpergewebe einzuführenden, bei Kontakt mit Körperflüssigkeit bakterizide bzw. mikrobizide Metallionen abgebenden, gewebeverträglichen medizinischen Gerätes, bestehend aus einem Polyurethan-Elastomer, in dessen Hauptkette zu weniger als 50 Gew.-% ein organisches Silikonpolymer mit einem Molekulargewicht von 500 bis 10.000 vorhanden ist, und aus einer Metallionen abgebenden Substanz, wobei man Polyol, organisches Silikonpolymer sowie Isocyanat miteinander reagieren läßt und danach in die gewünschte Form bringt, dadurch gekennzeichnet, daß man vor der Zugabe des Isocyanats eine gut wasserlösliche, Metallionen abgebende Substanz verwendet, diese als wäßrige Lösung mit einem Feststoffanteil an der Gesamtpolymermasse von 1 bis 15 Gew.-% zumischt und gemeinsam mit dem Polyol entwässert, ohne daß eine mechanische oder chemische Nachbehandlung erfolgt.

**Claims**

1. Tissue-compatible medical apparatus which is to be inserted into the body tissue and releases bactericidal or microbicidal metal ions on contact with body fluid, and which consists of a polyurethane elastomer, in the main chain of which an organic silicone polymer having a molecular weight of 500 to 10,000 is present to the extent of less than 50 % by weight, and of a substance which releases metal ions, characterised in that the substance which releases metal ions is embedded over the entire cross-section of the elastomer in spatially uniform distribution and in amounts of 1 to 15 % by weight, based on the total weight of the elastomer.

2. Process for the production of a tissue-compatible medical apparatus which is to be inserted into the body tissue and releases bactericidal or microbicidal metal ions on contact with body fluid, and which consists of a polyurethane elastomer, in the main chain of which an organic silicone polymer having a molecular weight of 500 to 10,000 is present to the extent of less than 50 % by weight, and of a substance which releases metal ions, the polyol, the organic silicone polymer and the isocyanate being reacted with one another and the product then being brought into the desired form, characterised in that, before addition of the isocyanate, a substance which releases metal ions is admixed, in an amount of 1 to 15 % by weight of the total polymer weight, in spatially uniform distribution as a powder having a particle size ≦ 50 μm and is dehydrated together with the polyol, without mechanical or chemical after-treatment taking place.

3. Process for the production of a tissue-compatible medical apparatus which is to be inserted into the body tissue and releases bactericidal or microbicidal metal ions on contact with body fluid, and which consists of a polyurethane polymer, in the main chain of which an organic silicone polymer having a molecular weight of 500 to 10,000 is present to the extent of less than 50 % by weight, and of a substance which releases metal ions, the polyol, organic silicone polymer and isocyanate being reacted with one another and the product then being brought into the desired form, characterised in that, before addition of the isocyanate, a readily water-soluble substance which releases metal ions is used, and this is admixed as an aqueous solution having a solids content of 1 to 15 % by weight of the total polymer weight and is dehydrated together with the polyol, without mechanical or chemical after-treatment taking place.

**Revendications**

1. Instrument médical à introduire dans le tissu corporel, compatible avec le tissu et cédant des ions métalliques bactéricides resp. microbicides quand il est en contact avec du liquide corporel, ledit instrument médical consistant en un élastomère de polyuréthanne, dans la chaîne fondamentale duquel se trouve dans un pourcentage en poids inférieur à 50% un polymère organique de silicone d'un poids moléculaire de 500 à 10000 et en une substance cédant des ions métalliques, caractérisé en ce que la substance cédant des ions métalliques est incluse dans toute la section de l'élastomère en répartition uniforme dans l'espace et en quantités de 1 à 15 % en poids par rapport à la masse totale de l'élastomère.

2. Procédé de fabrication d'un instrument médical à introduire dans le tissu corporel, ledit instrument étant compatible avec le tissu, cédant des ions métalliques bactéricides resp. microbicides quand il est en contact avec du liquide corporel, et consistant en un élastomère de polyuréthanne, dans la chaîne fondamentale duquel se trouve dans un pourcentage en poids inférieur à 50% un polymère organique de silicone d'un poids moléculaire de 500 à 10000, et en une substance cédant des ions métalliques, ledit procédé faisant réagir ensemble du polyol, un polymère organique de silicone ainsi qu'un isocyanate, qui reçoivent ensuite la forme souhaitée et étant caractérisé en ce que l'on mélange en répartition uniforme dans l'espace, avant d'ajouter l'isocyanate, une substance cédant des ions métalliques dans une proportion de 1 à 15 % en poids de la masse polymère totale sous forme de poudre d'une granulométrie inférieure ou égale à 50 $\mu$m et qu'on la déshydrate ensemble avec le polyol, sans post-traitement mécanique ou chimique.

3. Procédé de fabrication d'un instrument médical à introduire dans le tissu corporel, ledit instrument étant compatible avec le tissu, cédant des ions métalliques bactéricides resp. microbicides quand il est en contact avec du liquide corporel, et consistant en un élastomère de polyuréthanne, dans la chaîne fondamentale duquel se trouve dans un pourcentage en poids inférieur à 50% un polymère organique de silicone d'un poids moléculaire de 500 à 10000, et en une substance cédant des ions métalliques, ledit procédé faisant réagir ensemble du polyol, un polymère organique de silicone ainsi qu'un isocyanate, qui reçoivent ensuite la forme souhaitée, caractérisé en ce qu'avant d'ajouter l'isocyanate on utilise une substance cédant des ions métalliques qui est bien soluble dans l'eau, qu'on ajoute celle-ci au mélange sous forme d'une solution aqueuse avec une teneur de matières solides de 1 à 15 % en poids de la masse polymère totale et qu'on la déshydrate ensemble avec le polyol, sans post-traitement mécanique ou chimique.